(19) Europäisches Patentamt
European Patent Office
Office européen des brevets

(11) Veröffentlichungsnummer: **0 413 357 A2**

(12) **EUROPÄISCHE PATENTANMELDUNG**

(21) Anmeldenummer: 90115813.9

(22) Anmeldetag: **17.08.90**

(51) Int. Cl.⁵: **C07D 251/70**

(30) Priorität: 17.08.89 DD 331840
17.08.89 DD 331841

(43) Veröffentlichungstag der Anmeldung:
**20.02.91 Patentblatt 91/08**

(84) Benannte Vertragsstaaten:
**AT DE FR GB IT NL SE**

(71) Anmelder: **LEUNA-WERKE AG**

**DDR-4220 Leuna 3(DD)**

(72) Erfinder: **Tille, Anton, Dr. Dipl.-Chem.**
**Am Birkenwäldchen 1a**
**DD-4020 Halle(DD)**
Erfinder: **Voigt, Dietrich, Dr. Dipl.-Chem.**
**BL. 516**
**DD-4090 Halle-Neustadt(DD)**
Erfinder: **Baumann, Wolfgang**
**Göhlitzsch Nr. 6**
**DD-4220 Leuna(DD)**
Erfinder: **Beckhaus, Wolfgang, Dipl.-Ing.**
**Liebigstrasse 7**
**DD-4220 Leuna(DD)**
Erfinder: **Heynisch, Eberhard, Dipl.-Ing.**
**M.-Hill-Strasse 33**
**DD-4850 Weissenfels(DD)**
Erfinder: **Schröder, Lothar, Dr. Dipl.-Chem.**
**Kötzschauer Strasse 44**
**DD-4201 Spergau(DD)**
Erfinder: **Knebel, Herbert, Dr. Dipl.-Chem.**
**Ho-chi-Minh-Strasse 13**
**DD-7601 Leipzig(DD)**

(54) **Verfahren zur Herstellung von 1,5-Diacetyl-2,4-dioxohexahydro-1,3,5-triazin.**

(57) 1,5-Diacetyl-2,4-dioxohexahydro-1,3,5-triazin wird durch Acetylierung von 2,4-Dioxohexahydro-1,3,5-triazin in einer turbulent gemischten Suspension mit Essigsäureanhydrid hergestellt, indem die Reaktion bei 15 bis 40 °C (288 bis 313 K), in Anwesenheit eines Verdünnungsmittels bis 77 °C (350 K), durch Zugabe von Säure gestartet und adiabatisch bis zum Reaktionsende geführt wird, worauf das Acetylierungsgemisch gegebenenfalls mit Essigsäure oder essigsäurehaltigen prozeßeigenen Produkten verdünnt wird. In einer besonderen Ausführungsform wird ein Temperaturmaximum im Bereich von 90 bis 105 °C (363 bis 378 K) durch Wahl einer entsprechenden Starttemperatur von 15 bis 40 °C (288 bis 313 K) eingestellt. Das Endprodukt kann zur Erhöhung der Bleichwirkung von peroxidische Zusätze enthaltenden Waschmitteln eingesetzt werden.

EP 0 413 357 A2

## VERFAHREN ZUR HERSTELLUNG VON 1,5-DIACETYL-2,4-DIOXOHEXAHYDRO-1,3,5-TRIAZIN

Die Erfindung betrifft ein Verfahren zur Herstellung von 1,5-Diacetyl-2,4-dioxohexahydro-1,3,5-triazin (nachfolgend als DADHT bezeichnet) durch Acetylierung von 2,4-Dioxohexahydro-1,3,5-triazin (nachfolgend als DHT bezeichnet). DADHT reagiert mit Wasserstoffperoxid unter Bildung von Peressigsäure und kann deshalb zur Erhöhung der Bleichwirkung von Waschmitteln eingesetzt werden, die peroxidische Zusätze enthalten.

Die N-Acetylierung von Säureamiden wird im allgemeinen mit überschüssigem Essigsäureanhydrid in Gegenwart saurer oder basischer Katalysatoren durchgeführt. So ist bekannt, DHT mit einem 15-fachen Überschuß an Essigsäureanhydrid bei der Siedetemperatur des Reaktionsgemisches zu acetylieren (Coll. Czech. Chem. Commun. 27 (1962) 1562-67). Trotz erheblicher Reaktionszeiten werden hierbei jedoch nur unvollständig acetylierte Produkte erhalten, die eine zusätzliche Reinigung erfordern.

Es ist auch bekannt, die durch Schwefelsäure katalysierte Acetylierung von DHT mit einem geringen Überschuß an Essigsäureanhydrid in Gegenwart von Essigsäure (DD-A-241 414) und gegebenenfalls zusätzlich von nichtionogenen Tensiden (DD-A-241 415) vorzunehmen. Durch die Zugabe von Essigsäure und gegebenenfalls nichtionogenen Tensiden wird zwar eine Verbesserung der Rührfähigkeit der Suspension und damit eine Erhöhung der DADHT-Ausbeute erreicht, zur Erzielung ausreichender Reaktionsgeschwindigkeiten ist es aber erforderlich, den Katalysator Schwefelsäure in einer Menge von ca. 0,096 kg/kg, bezogen auf reines DHT (ca. 0,11 mol/mol, berechnet für 96 %ige Schwefelsäure) einzusetzen. Daraus resultiert aber wiederum, daß die Katalysatorsäure von der Aufarbeitung des Reaktionsgemisches neutralisiert oder durch Behandeln mit Wasser entfernt werden muß und demzufolge im Prozeß nicht mehr genutzt werden kann.

Es ist schließlich auch bekannt, Sulfoessigsäure als Katalysator zu verwenden, da sie während der Acetylierung ohnehin aus Schwefelsäure entsteht. Sie kann aus Schwefelsäure und Essigsäureanhydrid vorgebildet werden (DD-A-229 696). Nach diesem Verfahren werden aber nur dunkel gefärbte Lösungen von Sulfoessigsäure erhalten, deren Einsatz in der Acetylierung von DHT demzufolge auch zu dunkel gefärbtem, einer Nachreinigung zu unterziehendem DADHT führt. Deshalb ist es vorteilhafter, die Sulfoessigsäure im Acetylierungsgemisch selbst zu erzeugen (DD-A-253 423). Die bei der Abtrennung des DADHT anfallende sulfoessigsäurehaltige Mutterlauge kann zur Erhöhung der Katalysatorkonzentration in die Reaktionsstufe zurückgeführt werden. Da aber Sulfoessigsäure im Vergleich zur Schwefelsäure katalytisch wesentlich weniger wirksam ist, müssen selbst bei Sulfoessigsäurekonzentrationen von 0,08 bis 0,12 mol/mol, bezogen auf DHT, zusätzlich bis zu 3 l 96 %iger Schwefelsäure je 110 kg 89- bis 92 %iges DHT, entsprechend 0,063 mol/mol (berechnet mit $d_{H2SO4} = 1,84$ g/cm$^3$ für durchschnittlich 90 %iges DHT und bezogen auf reines DHT) zugegeben, Reaktionstemperaturen bis 115 °C (388 K) und Reaktionszeiten bis zu 6 Stunden eingehalten werden. Durch die langen Reaktionszeiten bei hohen Temperaturen und hohen Säurekonzentrationen wird aber die Bildung gefärbter Nebenprodukte begünstigt, die direkt oder im Ergebnis der Kreislaufführung der Mutterlauge in das DADHT gelangen und dessen Qualität mindern.

In den genannten Verfahren ist der Einsatz von Essigsäure als Verdünnungsmittel besonders nachteilig, da Essigsäure als Reaktionsprodukt der Gleichgewichtsreaktion entsteht und demzufolge jede Erhöhung ihrer Konzentration im Acetylierungsgemisch der Umsetzung von DHT zu DADHT entgegenwirkt. Die Anwesenheit eines Verdünnungsmittels ist nach diesem Verfahren aber notwendig, um sowohl eine ausreichende Rührfähigkeit als auch eine Begrenzung des als Folge der hohen Exothermie der Reaktion (ca. 75 kJ/mol) auftretenden adiabatischen Temperatursprungs zu sichern. Daraus resultiert aber wiederum, daß die vorgesehene Endtemperatur zumindest durch Nachheizen des Acetylierungsgemisches (DD-A-241 414) oder durch ein kompliziertes Regime der Zugabe von Essigsäure oder Mutterlauge (DD-A-253 423) eingestellt werden muß.

Der Erfindung liegt die Aufgabe zugrunde, ein Verfahren zur Herstellung von 1,5-Diacetyl-2,4-dioxohexahydro-1,3,5-triazin durch Umsetzung von DHT mit Essigsäureanhydrid in Gegenwart einer Säure als Katalysator anzugeben, das mit hoher Reaktionsgeschwindigkeit, hoher Raum-Zeit-Ausbeute und niedrigem Energieaufwand so durchführbar ist, daß die Reaktion in kurzer Zeit praktisch quantitativ zu Ende geführt und ein gut filtrierbarer Feststoff abgeschieden wird und die thermische Belastung des Reaktionsgemisches gering bleibt. Weiterhin soll der Anteil der Nebenprodukte bei diesem Verfahren gering bleiben.

Die Aufgabe wird anspruchsgemäß gelöst. Die abhängigen Ansprüche betreffen vorteilhafte Ausführungsformen.

Das erfindungsgemäße Verfahren zur Herstellung von 1,5-Diacetyl-2,4-dioxohexahydro-1,3,5-triazin durch Umsetzen von 2,4-Dioxohexahydro-1,3,5-triazin mit Essigsäureanhydrid in einer turbulent gerührten Mischung und in Gegenwart einer Säure als Katalysator ist dadurch gekennzeichnet, daß die Reaktion bei Temperaturen im Bereich von 15 bis 40 °C (288 bis 313 K), in Anwesenheit eines Verdünnungsmittels bis 77 °C (350 K), durch Zugabe der Säure gestartet und adiabatisch weitergeführt und das Acetylierungsgemisch nach Erreichen des Temperaturmaximums gegebenenfalls auf 110 bis 150 % seiner verdünnungsmittelfreien Masse mit Essigsäure und/oder essigsäurehaltigen prozeßeigenen Produkten verdünnt wird.

Das Verfahren kann in Rührgefäßen bekannter Bauart mit Einrichtungen zur Kühlung und Beheizung realisiert werden, wenn eine turbulente Durchmischung gesichert ist. Während der Acetylierung bleibt das Gemisch rührfähig. Das ist insofern überraschend, da bekannt ist (DD-A-241 414 und DD-A-241 415), daß die Acetylierung von DHT mit Essigsäureanhydrid in Gegenwart von Schwefelsäure und bei Abwesenheit eines Verdünnungsmittels bereits bei Starttempera turen von 40 °C zu viskos-pastösen Reaktionsgemischen führt und demzufolge DADHT-Ausbeuten von lediglich 63 % erreicht werden können.

Im allgemeinen kann die Reaktion bei Raumtemperatur gestartet werden. Sie ist in Abhängigkeit von den Wärmeverlusten des Reaktionssystems nach einem adiabatischen Temperatursprung von 60 bis 85 K beendet. Ein Aufheizen des Einsatzgemisches ist nur dann erforderlich, wenn durch extreme Wärmeverluste des Reaktionssystems für eine ausreichende Reaktionsgeschwindigkeit zweckmäßige Temperaturen nicht erreicht werden. Nach einer besonderen Ausgestaltung des Verfahrens ist es dann vorteilhaft, daß im Acetylierungsgemisch ein Temperaturmaximum im Bereich von 90 bis 105 °C (363 bis 378 K) durch eine entsprechende Starttemperatur im Bereich von 15 bis 40 °C (288 bis 313 K) eingestellt wird.

Eine weitere vorteilhafte Ausgestaltung des erfindungsgemäßen Verfahrens besteht darin, daß für die als Katalysator zu verwendende Säure oberhalb einer Mindestkonzentration von 0,08 mol je kg Reaktionsgemisch eine Menge von 0,6 bis 1,0 Äquivalenten, bezogen auf die Menge der im 2,4-Dioxohexahydro-1,3,5-triazin enthaltenen protonierbaren Verbindungen, eingehalten wird und die Zugabe der Säure innerhalb eines Temperaturbereiches von 15 bis 77 °C (288 bis 350 K) bei einer Temperatur erfolgt, die gemäß der Gleichung

$$T = 443 - 155 \cdot SÄ ,$$

in der T die Temperatur in K und SÄ die Anzahl der Säureequivalente bedeuten, berechnet wird.

Dabei werden die Konzentrationsverhältnisse in dem Acetylierungsgemisch so gewählt, daß die durch die Zugabe der Säure ausgelöste exotherme Reaktion zwischen DHT und Essigsäureanhydrid einen adiabatischen Temperaturanstieg von 1,0 bis maximal 5,0 K/min und vorzugsweise von 1,5 bis 3,0 K/min verursacht. Dieses Ziel kann sowohl bei niedrigen Starttemperaturen durch den Verzicht auf ein Verdünnungsmittel, wie beispielsweise Essigsäure und/oder Mutterlauge bzw. prozeßeigene Verdünnungsmittel und/oder Waschfiltrate, als auch bei höheren Starttemperaturen in Gegenwart eines Verdünnungsmittels erreicht werden.

Nach dem erfindungsgemäßen Verfahren wird die Acetylierung des DHT schnell zu Ende geführt. Der quantitative Umsatz des DHT ist praktisch mit dem Erreichen des Temperaturmaximums erzielt. Es kann aber zweckmäßig sein, die Reaktionszeit über das Temperaturmaximum der exothermen Reaktion hinaus nochmals um maximal die Zeit zu verlängern, die sie vom Start bis zum Erreichen des Temperaturmaximums benötigt hat.

Als Katalysatoren nach dem erfindungsgemäßen Verfahren verwendbare Säuren sind alle diejenigen, die in einem wasserfreien Medium, wie Essigsäureanhydrid oder Essigsäureanhydrid/Essigsäure-Mischungen, ausreichend disoziieren, also beispielsweise Perchlorsäure und Schwefelsäure. Dabei ist zu beachten, daß das Säureequivalent der in wäßrigen Systemen mehrbasigen Säuren unter Acetylierungsbedingungen nur 1 beträgt.

An die Qualität des einzusetzenden DHT werden keine besonderen Forderungen gestellt. Verunreinigungen wie Harnstoff, Biuret, Methylenbis(harnstoff) und Cyanursäure, die in technischen Produkten stets enthalten sind, stören die Acetylierung zumindest bis zu einem Gehalt von 0,15 kg/kg nicht. Da Cyanursäure nach jedem bekannten Verfahren zwangsläufig zu einem erheblichen Anteil in das Zielprodukt gelangt, sollte ihr Gehalt im DHT 0,10 kg/kg nicht überschreiten, damit ein DADHT mit weniger als 0,05 kg/kg Cyanursäure erhalten werden kann. Außerdem ist es vorteilhaft, wenn das DHT weitgehend frei von säureverbrauchenden Verunreinigungen ist; zumindest sollte aber ein Säureverbrauch von 0,1 mol Säure je 1 kg DHT nicht überschritten werden. Der Säureverbrauch des DHT wird durch potentiometrische Titration einer Probe des DHT mit Perchlorsäure, eventuell auch Schwefelsäure, in wasserfreier Essigsäure nach bekannten Verfahren ermittelt. Der Säureverbrauch des DHT wird in Mol Säure je Kilogramm DHT angegeben.

Als Essigsäureanhydrid wird bei dem erfindungsgemäßen Verfahren ein technisches Produkt mit einem Gehalt von ca. 0,94 kg/kg Essigsäureanhydrid verwendet. Die bei der Acetylierung eingesetzte Essigsäureanhydridmenge muß mindestens allen Essigsäureanhydrid verbrauchenden Prozessen entsprechen. Auf jeden Fall genügt für die Acetylierung des DHT ein 5 %iger Überschuß an Essigsäureanhydrid (2,1 mol Essigsäureanhydrid je Mol DHT). Bei der Festlegung der Einsatzmenge an Essigsäureanhydrid ist aber der Gehalt an ebenfalls acetylierbaren Verunreinigungen im DHT zu berücksichtigen.

In Abhängigkeit von der Qualität des eingesetzten DHT sind Schwefelsäuremengen von 0,03 bis 0,07 mol/mol, bezogen auf DHT, ausreichend, um selbst bei einer Starttemperatur von 15 °C (288 K) die Acetylierung in maximal 75 min zu beenden. Als Verdünnungsmittel wird am einfachsten Essigsäure eingesetzt. Dabei verbleibt aber ein der Löslichkeit entsprechender Anteil DADHT in Lösung. Vorteilhafter ist es deshalb, bei der Abtrennung des DADHT anfallende Mutterlauge und/oder Waschfiltrate zu verwenden, da diese Produkte bereits mit DADHT gesättigt sind und demzufolge vergleichsweise höhere Ausbeuten an DADHT erreicht werden können. Selbstverständlich ist es auch möglich, die genannten Verdünnungsmittel in beliebigem Verhältnis nacheinander oder als Gemisch zuzugeben. Durch die Zugabe des Verdünnungsmittels wird die Temperatur des Acetylierungsgemischs um 10 bis 25 K gesenkt.

Das nach der Zugabe des Verdünnungsmittels resultierende Reaktionsgemisch kann nach beliebigen bekannten Verfahren aufgearbeitet werden. Am einfachsten wird zunächst auf Temperaturen im Bereich von 20 bis 40 °C (293 bis 313 K) abgekühlt. Dabei bleibt die Suspension selbst bei Zugabe von nur 0,10 kg Verdünnungsmittel je Kilogramm des Acetylierungsgemisches zumindest bis zu einer Temperatur von 20 °C (293 K) rührfähig. Beim Vorliegen spezieller Transportprobleme kann die Menge an Verdünnungsmittel natürlich erhöht werden. Doch ist es weder notwendig noch wegen der zu empfehlenden Aufarbeitung der Mutterlauge und der Waschfiltrate zweckmäßig, die Menge des Verdünnungsmittels auf mehr als 0,50 kg je Kilogramm des Acetylierungsgemisches zu steigern. Aus der abgekühlten Suspension wird das auskristallisierte DADHT z.B. durch Filtration oder Zentrifugieren abgetrennt, mit Essigsäure gewaschen und getrocknet. Bei geringeren Forderungen an die Reinheit des DADHT kann auf das Waschen mit Essigsäure verzichtet werden. Die Mutterlauge und die Waschfiltrate werden erfindungsgemäß als Verdünnungsmittel eingesetzt. Aus überschüssigen Mengen an Mutterlauge und Waschfiltraten werden nach bekannten Verfahren Essigsäure und Essigsäureanhydrid abgetrennt. Aus den Sumpfprodukten können weitere Mengen an DADHT gewonnen werden. Es ist aber auch möglich, nach bekannten Verfahren die im Reaktionsgemisch enthaltene Katalysatorsäure zu neutralisieren, die Essigsäure vollständig oder teilweise abzudestillieren, den Rückstand in Wasser aufzunehmen und durch geeignete Maßnahmen das DADHT aus der wäßrigen Suspension zu gewinnen.

Ebenso ist es möglich, durch Zugabe von Wasser zum Reaktionsgemisch eine Suspension mit einem Feststoffanteil von 0,20 bis 0,35 kg/kg herzustellen und das nach Fest-Flüssig-Trennung erhaltene DADHT zu trocknen.

Das erfindungsgemäße Verfahren wird im folgenden anhand von Ausführungsbeispielen näher erläutert.

Beispiele 1 bis 5

Die Acetylierung wurde in einem Dewar-Gefäß mit einem inneren Durchmesser von 80 mm durchgeführt. Das Dewar-Gefäß wurde mit einem Deckel verschlossen, der als Halterung für verschiedene Zusatzeinrichtungen diente, die in das Gefäß hineinragten (Rührer, Katalysatordosierer, Heizstab, Kühlfinger, Thermoelement) bzw. aufgesetzt waren (Tropftrichter). Das Dewar-Gefäß wurde mit DHT und Essigsäureanhydrid, der Katalysatordosierer mit Schwefelsäure beschickt. Durch Betrieb des Heizstabes und/oder des Kühlfingers wurden bei turbulentem Durchmischen DHT und Essigsäureanhydrid und die im Katalysatordosierer befindliche Schwefelsäure auf die vorgesehene Starttemperatur gebracht. Danach wurden Heizstab und/oder Kühlfinger außer Betrieb gesetzt und die Reaktion durch Zerschlagen des Bodens des Katalysatordosierers mittels eines darin installierten Hilfsrührers gestartet. Nach Erreichen der Maximaltemperatur wurde aus dem Tropftrichter das Verdünnungsmittel zugegeben und das Reaktionsgemisch durch Inbetriebnahme des Kühlfingers auf die vorgesehene Temperatur für die Fest-Flüssig-Trennung abgekühlt. Das kristalline DADHT wurde mittels einer R4-Fritte unter vermindertem Druck von der Mutterlauge abgetrennt, zweimal mit je 100 ml Essigsäure und jeweils entsprechender Abtrennung der Waschfiltrate gewaschen und bei maximal 117 °C (390 K) getrocknet. Mutterlauge und Waschfiltrate wurden als Verdünnungsmittel eingesetzt. Versuchsparameter, Reaktionsdaten und Angaben zum Reaktionsprodukt sind in Tabelle 1 zusammengestellt.

Beispiel 6

Die Acetylierung wurde in einem zylinderförmigen Reaktionsgefäß mit einem inneren Durchmesser von 80 mm durchgeführt, das gegen Wärmeverluste isoliert war. Deckel, Zusatzeinrichtungen und Arbeitsweise entsprechen den Beispielen 1 bis 5.

4

Versuchsparameter, Reaktionsdaten und Angaben zum Reaktionsprodukt sind in Tabelle 1 enthalten.

Beispiele 7 und 8

Die Acetylierung wurde in einem ummantelten und isolierten emaillierten Rührwerk mit einem Nenninhalt von 630 l durchgeführt, das mit Impeller-Rührer und Strombrecher sowie Einrichtungen zur Produktzuführung und -abführung und zur Temperaturmessung ausgerüstet war. In dem Rührwerk wurden DHT und Essigsäureanhydrid bei turbulentem Durchmischen und Temperieren des Mantelraumes mit Warmwasser auf die vorgesehene Starttemperatur gebracht und die Acetylierung nach Außerbetriebnahme der Manteltemperierung durch Zugabe von Schwefelsäure eingeleitet. Nach Erreichen der Maximaltemperatur wurde das Verdünnungsmittel zugegeben und das Reaktionsgemisch durch Beschicken des Mantelraumes mit Kühlwasser auf die vorgesehene Temperatur für die Fest-Flüssig-Trennung abgekühlt. Das kristalline DADHT wurde mit einer Zentrifuge von der Mutterlauge abgetrennt, zweimal mit je 50 l Essigsäure und jeweils entsprechender Abtrennung der Waschfiltrate gewaschen und in einem Vakuumschaufeltrockner getrocknet. Die Mutterlauge wurde als Verdünnungsmittel eingesetzt.

Versuchsparameter, Reaktionsdaten und Angaben zum Reaktionsprodukt sind in Tabelle 2 zusammengestellt.

Tabelle 1

| Beispiel | 1 | 2 | 3 | 4 | 5 | 6 |
|---|---|---|---|---|---|---|
| **Einsatzprodukte** | | | | | | |
| DHT | | | | | | |
| Menge (g) | 135,0 | 139,0 | 135,0 | 135,0 | 135,0 | 135,0 |
| Gehalt (kg/kg) | 0,900 | 0,900 | 0,900 | 0,880 | 0,995 | 0,880 |
| Essigsäureanhydrid | | | | | | |
| Menge (g) | 259,0 | 255,5 | 260,0 | 258,0 | 261,5 | 259,0 |
| Gehalt (kg/kg) | 0,950 | 0,950 | 0,950 | 0,950 | 0,950 | 0,950 |
| Schwefelsäure | | | | | | |
| Menge (g) | 6,0 | 5,5 | 5,0 | 7,0 | 3,5 | 6,0 |
| Gehalt (kg/kg) | 0,960 | 0,960 | 0,960 | 0,960 | 0,960 | 0,960 |
| **Verdünnungsmittel** | | | | | | |
| Essigsäure | | | | | | |
| Menge (g) | | | 150,0 | | | |
| Gehalt (kg/kg) | | | 0,995 | | | |
| Mutterlauge | | | | | | |
| Menge (g) | 160,0 | 200,0 | | | 40,0 | 100,0 |
| VR [a] (kg/kg) | 0,117 | 0,117 | | | 0,117 | 0,117 |
| DADHT-Gehalt (kg/kg) | 0,038 | 0,038 | | | 0,038 | 0,038 |
| Waschfiltrat | | | | | | |
| Menge (g) | | | | 100,0 | | |
| VR (kg/kg) | | | | 0,091 | | |
| DADHT-Gehalt | | | | 0,029 | | |
| **Reaktionsdaten** | | | | | | |
| Starttemperatur ($^\circ$C) | 25 | 25 | 30 | 32 | 15 | 40 |
| Starttemperatur (K) | 298 | 298 | 303 | 305 | 288 | 313 |
| Maximaltemperatur ($^\circ$C) | 100 | 99 | 103 | 105 | 100 | 90 |
| Maximaltemperatur (K) | 373 | 372 | 376 | 378 | 373 | 363 |
| Reaktionszeit (min) | 61 | 72 | 70 | 45 | 75 | 75 |
| Temperatur ($^\circ$C) | 30 | 20 | 30 | 40 | 30 | 30 |
| Fest-Flüssig-Trennung (K) | 303 | 293 | 303 | 315 | 303 | 303 |
| **Reaktionsprodukt** | | | | | | |
| Menge (g) | 203,1 | 203,9 | 196,0 | 190,2 | 218,0 | 196,2 |
| DADHT-Gehalt (kg/kg) | 0,952 | 0,950 | 0,956 | 0,958 | 0,990 | 0,949 |
| PWG[b] (%) | 89 | 89 | 90 | 88 | 90 | 89 |
| Ausbeute (mol/mol) | 0,920 | 0,921 | 0,891 | 0,886 | 0,928 | 0,906 |

[a] Verdampfungsrückstand
[b] Pulverweißgrad

Tabelle 2

| Beispiel | 7 | 8 |
|---|---|---|
| **Einsatzprodukte** | | |
| DHT | | |
| Menge (kg) | 130 | 130 |
| Gehalt (kg/kg) | 0,900 | 0,880 |
| Essigsäureanhydrid | | |
| Menge (g) | 345 | 240 |
| Gehalt (kg/kg) | 0,940 | 0,940 |
| Schwefelsäure | | |
| Menge (kg) | 5,5 | 6,0 |
| Gehalt (kg/kg) | 0,960 | 0,960 |
| **Verdünnungsmittel** | | |
| Mutterlauge | | |
| Menge (kg) | 95 | 135 |
| VR[a]) (kg/kg) | 0,160 | 0,160 |
| DADHT-Gehalt(kg/kg) | 0,040 | 0,040 |
| **Reaktionsdaten** | | |
| Starttemperatur (°C) | 25 | 30 |
| Starttemperatur (K) | 298 | 303 |
| Maximaltemperatur (°C) | 98 | 101 |
| Maximaltemperatur (K) | 371 | 374 |
| Reaktionszeit (min) | 52 | 48 |
| Temperatur (°C) | 30 | 35 |
| Fest-Flüssig-Trennung (K) | 303 | 308 |
| **Reaktionsprodukt** | | |
| Menge (kg) | 194 | 189 |
| DADHT-Gehalt(kg/kg) | 0,950 | 0,946 |
| PWG[b]) (%) | 88 | 88 |
| Ausbeute (mol/mol) | 0,910 | 0,903 |

[a])Verdampfungsrückstand
[b])Pulverweißgrad

Beispiele 9 bis 15

In einem beheiz- und kühlbaren, mit einem Doppelmantel und Einrichtungen zur Temperaturkontrolle sowie zum Dosieren von Flüssigkeiten und Feststoffen ausgerüsteten Rührwerk mit einem Nutzvolumen von 600 l und einem Impellerrührer zum intensiven Durohmischen aller Reaktanten wurden zunächst das Essigsäureanhydrid und, wenn vorgesehen (vgl. Tabelle 3), die Mutterlauge oder Essigsäure und danach das DHT-Pulver vorgelegt. Die gerührte Mischung von DHT in Essigsäureanhydrid und eventuell Mutterlauge oder Essigsäure wurde auf die vorgegebene Starttemperatur aufgeheizt; nach dem Erreichen der Starttemperatur wurde die vorgesehene Menge Säure innerhalb von maximal 5 Minuten zugegeben. Nach der Dosierung der Säure begann die Acetylierungsreaktion, erkennbar an einem schnellen Temperaturanstieg ohne äußere Wärmezufuhr. Nach dem Erreichen des Temperaturmaximums war die Umsetzung des DHT mit dem Essigsäureanhydrid praktisch abgeschlossen. Um eventuell vorhandene gröbere Bestandteile des DHT ebenfalls vollständig umzusetzen, schloß sich an das Temperaturmaximum noch eine Nachreaktionsphase an, während der die Temperatur des Reaktionsgemisches bereits wieder zu sinken begann. Nach Abschluß der Nachreaktionsphase wurde, falls dies vorgesehen war (vgl. Tabelle 3), zum Verdünnen des Kristallbreis Mutterlauge oder Waschfiltrat zugesetzt und anschließend das Acetylierungsgemisch auf

## EP 0 413 357 A2

35 ± 2 °C (308 ± 2 K) abgekühlt, um das DADHT abzuscheiden.

Die Isolierung des DADHT erfolgte mit einer Zentrifuge. Der Filterkuchen wurde mit Essigsäure gewaschen und der essigsäurefeuchte Filterkuchen im Vakuum getrocknet. Die Reaktionsbedingungen und Ergebnisse der Ausführungsbeispiele sind in der Tabelle 3 zusammengestellt. Die analytische Charakterisierung des DADHT erfolgte durch Ermittlung des DADHT-, Cyanursäure- und DHT-Gehaltes mittels HPLC und des Pulverweißgrades mit einem Leukometer.

Tabelle 3

| Beispiel | 9 | 10 | 11 | 12 | 13 | 14 | 15 |
|---|---|---|---|---|---|---|---|
| DHT Menge (kg) | 131 | 131 | 129 | 129 | 130 | 130 | 130 |
| Gehalt DHT(kg/kg) | 0,866 | 0,866 | 0,774 | 0,926 | 0,884 | 0,884 | 0,884 |
| Gehalt CNS(kg/kg) | 0,109 | 0,109 | 0,165 | 0,073 | 0,103 | 0,103 | 0,103 |
| Säureverbrauch (mol $H^+$/kg) | 0,47 | 0,47 | 0,94 | 0,24 | 0,39 | 0,39 | 0,39 |
| Essigsäureanhydrid 94%ig, (kg) | 254 | 254 | 254 | 240 | 254 | 254 | 254 |
| Säure als Katalysator Art | $H_2SO_4$ | $HClO_4$ | $H_2SO_4$ | $H_2SO_4$ | $H_2SO_4$ | $H_2SO_4$ | $H_2SO_4$ |
| Menge (kg) | 4,5 | 5,6 | 11,0 | 3,2 | 5,0 | 5,6 | 4,8 |
| vor dem Start zugesetzte Essigsäure(kg) | - | - | - | - | - | - | 80 |
| Mutterlauge(kg) | 20 | - | 20 | - | 80 | 180 | - |
| Verhältnisse Säure/Basizität (mol) | 0,72 | 0,65 | 0,89 | 1,00 | 0,96 | 0,95 | 0,93 |
| Säure(Reaktionsgemisch mol/kg) | 0,11 | 0,10 | 0,28 | 0,08 | 0,10 | 0,10 | 0,13 |
| Acetylierungsbedingungen | | | | | | | |
| Starttemperatur (°C) | 30 | 32 | 28 | 25 | 60 | 70 | 60 |
| Starttemperatur (K) | 303 | 305 | 301 | 298 | 333 | 343 | 338 |
| Temperatursteigerung (K/min) | 2,6 | 2,0 | 2,8 | 3,0 | 1,8 | 1,0 | 1,3 |
| Temperaturmaximum (°C) | 108 | 106 | 107 | 102 | 115 | 106 | 116 |
| Temperaturmaximum (K) | 381 | 379 | 380 | 275 | 388 | 379 | 389 |
| Reaktionszeit (min) bis Temperaturmaximum gesamt (min) | 30 | 38 | 28 | 26 | 30 | 40 | 38 |
| | 60 | 70 | 50 | 50 | 60 | 75 | 75 |
| Isolierung des DADHT Zugabe von Mutterlauge/Waschfiltrat* nach Gesamtreaktionszeit (kg) | 160 | 180 | 150 | 150 | 100 | - | 70 |
| Abkühlen auf (°C) | 35 | 36 | 35 | 35 | 34 | 36 | 35 |
| Abkühlen auf (K) | 308 | 309 | 308 | 308 | 307 | 309 | 308 |
| Ausbeute DADHT (kg) | 178 | 180 | 176 | 188 | 185 | 178 | 175 |
| mol/mol bez. auf DHT | 0,850 | 0,851 | 0,900 | 0,861 | 0,860 | 0,835 | 0,826 |
| isoliertes DADHT | | | | | | | |
| Gehalt an DADHT(kg/kg) | 0,936 | 0,928 | 0,881 | 0,948 | 0,926 | 0,934 | 0,940 |
| Gehalt CNS (kg/kg) | 0,059 | 0,054 | 0,104 | 0,034 | 0,060 | 0,058 | 0,046 |
| Gehalt DHT (kg/kg) | 0,003 | 0,004 | 0,003 | 0,002 | 0,003 | 0,002 | 0,002 |
| Pulverweißgrad (%) | 89 | 90 | 88 | 90 | 87 | 88 | 90 |

\* Beispiel 12 = Waschfiltrat

Anmerkungen: CNS = Cyanursäure
Konzentration der Säuren:
$H_2SO_4$ 0,96 kg/kg
$HClO_4$ 0,72 kg/kg.

**Ansprüche**

1. Verfahren zur Herstellung von 1,5-Diacetyl-2,4-dioxohexahydro-1,3,5-triazin durch Umsetzung von 2,4-Dioxohexahydro-1,3,5-triazin mit Essigsäureanhydrid in einer turbulent gerührten Mischung und in Gegenwart einer Säure als Katalysator,
dadurch **gekennzeichnet**,
daß die Reaktion bei Temperaturen im Bereich von 15 bis 40 °C (288 bis 313 K), bei Anwesenheit eines Verdünnungsmittels bei Temperaturen bis 77 °C (350 K), durch Zugabe der Säure gestartet und adiabatisch weitergeführt wird.

2. Verfahren nach Anspruch 1, dadurch gekennzeichnet, daß das Acetylierungsgemisch nach Erreichen des Temperaturmaximums auf 110 bis 150 % seiner verdünnungsmittelfreien Masse mit Essigsäure und/oder essigsäurehaltigen prozeßeigenen Produkten verdünnt wird.

3. Verfahren nach Anspruch 1 oder 2, dadurch gekennzeichnet, daß im Acetylierungsgemisch ein Temperaturmaximum im Bereich von 90 bis 105 °C (363 bis 378 K) durch eine Starttemperatur zwischen 15 und 40 °C (288 bis 313 K) eingestellt wird.

4. Verfahren nach einem der Ansprüche 1 bis 3, dadurch gekennzeichnet, daß für die als Katalysator verwendete Säure oberhalb einer Mindestkonzentration von 0,08 mol/kg Reaktionsgemisch eine Menge von 0,6 bis 1,0 Äquivalenten, bezogen auf die Menge der im 2,4-Dioxohexahydro-1,3,5-triazin enthaltenen protonierbaren Verbindungen, eingehalten wird und die Zugabe der Säure innerhalb eines Temperaturbereiches von 15 bis 77 °C (288 bis 350 K) bei einer Temperatur erfolgt, die gemäß der Gleichung T = 443 - 155 • SÄ, in der T die Temperatur in K und SÄ die Anzahl der Säureäquivalente bedeuten, berechnet wird.

5. Verfahren nach einem der Ansprüche 1 bis 4, dadurch gekennzeichnet, daß als Katalysator Schwefelsäure oder Perchlorsäure verwendet wird.

6. Verfahren nach einem der Ansprüche 1 bis 5, dadurch gekennzeichnet, daß die Reaktionsdauer maximal das Doppelte der Zeit vom Start der Reaktion bis zum Erreichen des Temperaturmaximums beträgt.